(19) Europäisches Patentamt
European Patent Office
Office européen des brevets

(11) Numéro de publication: 0 152 319
B1

(12) # FASCICULE DE BREVET EUROPÉEN

(45) Date de publication du fascicule du brevet: 22.07.87

(51) Int. Cl.[4]: C 07 C 69/734, C 07 C 67/343

(21) Numéro de dépôt: 85400100.5

(22) Date de dépôt: 23.01.85

(54) **Procédé d'obtention de composés organiques à groupe alkoxyalkylidène.**

(30) Priorité: 07.02.84 FR 8402027

(43) Date de publication de la demande: 21.08.85 Bulletin 85/34

(45) Mention de la délivrance du brevet: 22.07.87 Bulletin 87/30

(84) Etats contractants désignés: AT BE CH DE FR GB IT LI LU NL SE

(56) Documents cité: FR-A-2 273 793

(73) Titulaire: RHONE- POULENC SPECIALITES CHIMIQUES, "Les Miroirs" 18, Avenue d'Alsace, F-92400 Courbevoie (FR)

(72) Inventeur: Ratton, Serge, Vaulx- Milieu, F-38290 - La Verpillière (FR)

(74) Mandataire: Cazes, Jean- Marie, RHONE- POULENC INTERSERVICES Service Brevets Chimie 25, quai Paul Doumer, F-92408 Courbevoie Cédex (FR)

EP 0 152 319 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1987

**Description**

La présente invention a pour objet un procédé d'obtention de composés à groupe alkoxyalkylidène, et plus particulièrement un procédé d'obtention d'alkoxyalkylidènemalonates d'alkyle par condensation d'un orthoester avec un malonate d'alkyle.

Les composés organiques qui comportent un groupe alkoxyalkylidène répondant à la formule générale:

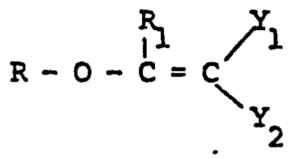

dans laquelle R represente un radical alkyle, $R_1$ un atome d'hydrogène, un radical alkyle ou un groupe phényle et $Y_1$ et $Y_2$, identiques ou différents représentent un reste alkyloxycarbonyle, nitrile, alkylcarbonyle, sont des intermédiaires particulièrement recherchés en synthèse organique. Ainsi les alkoxyméthylènemalonates d'alkyle sont utilisés pour l'obtention d'anilinométhylènemalonates d'alkyle qui sont des intermédiaires de synthèse des quinoléïnes substituées telles que la dichloro-4,7 quinoléïne ou la chloro-4 trifluorométhyl-7 quinoléïne (cf. brevet français 950.883).

Les alkoxyalkylidènemalonates d'alkyle sont obtenus par condensation d'un malonate d'alkyle (malonate d'éthyle par exemple) avec un orthoester: orthoformiates, orthoacétates, orthobenzoates d'alkyle et en particulier de méthyle ou d'éthyle, cf. L. CLAISEN, Ber. 26 page 2729 et suivantes (1893) et Ann. 297 page 16 et suivantes (1897); P. SAH, J. Am. Chem. Soc. 53 page 1836 et suivantes (1931). Cette condensation qui peut être représentée par le schéma réactionnel suivant:

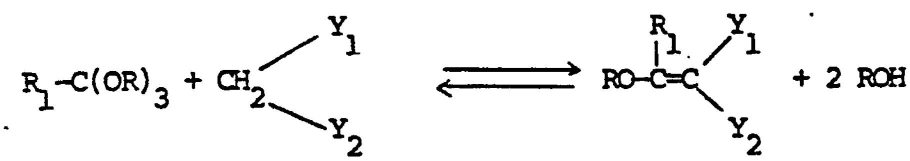

est en général conduite en présence de chlorure de zinc comme catalyseur et d'anhydride acétique comme agent de condensation. Cette reaction a fait l'objet de diverses études mécanistiques [cf. H. W. POST et al, J. Org. Chem. 2 page 260 et suivantes (1937) et R. C. FUSON et al, J. Org. Chem. 11 page 194 et suivantes (1946)] notamment dans le but d'améliorer les rendements en alkoxyméthylènemalonates (cf. R. C. FUSON et al, loc. cit.). Par la suite on a proposé divers perfectionnements au procédé de CLAISEN dans le but d'élever les rendements en alkoxyméthylènemalonates d'alkyle (en particulier vis-à-vis des orthoformiates) et la productivité de la réaction. Dans le brevet américain 2.824.121, on a proposé de réaliser la condensation en absence de chlorure de zinc, de remplacer l'anhydride acétique par une quantité d'acide acétique insuffisante pour bloquer sous forme d'acétate l'alcool formé au cours de la réaction et d'éliminer ce dernier par distillation au fur et à mesure de sa formation. En dépit de l'amélioration des résultats qu'il a procuré, ce procédé n'a pas conduit à des rendements suffisants vis-à-vis de l'orthoformiate. Dans la demande de brevet japonais, publiée sous le No. 4776/66, on a préconisé d'opérer en présence de catalyseurs métalliques (sels de zinc et de fer) en absence d'acide ou d'anhydride, au sein d'un solvant hydrocarboné (benzène, xylène, toluène) en assurant l'élimination de l'alcool formé par distillation azeotropique. Les rendements attribués à ce procédé n'ont pu être confirmés. Enfin dans la demande de brevet français No. 75/17.177, publiée sous le No. 2.273.793 on a proposé de préparer les alkoxyméthylènemalonates d'alkyle en faisant réagir un malonate d'alkyle avec un excès d'orthoformiate d'alkyle à 100-160°C, en présence d'un sel de zinc, d'aluminium ou de fer et en éliminant l'alcool résultant de la condensation au fur et à mesure de sa formation. Malgré l'amélioration des rendements qu'il apporte, ce procédé ne permet pas de dépasser un rendement théorique en alkoxyméthylènemalonate de 90 % par rapport à l'orthoformiate de sorte que l'industrie est encore à la recherche de moyens permettant d'augmenter ce rendement. Les orthoformiates constituent en effet une matière première coûteuse et toute amélioration de leur transformation en alkoxyméthylènemalonates entraîne une diminution sensible de leur prix de revient.

Un des objets de la présente invention réside précisément dans l'amélioration des rendements en alkoxyalkylènemalonates vis-à-vis des orthoesters et accessoirement vis-à-vis des malonates. Un autre objet de la présente invention réside dans l'élargissement du choix des catalyseurs de condensation.

Plus spécifiquement la présente invention a pour objet un procédé de préparation d'alkoxyalkylidènemalonates d'alkyle de formule:

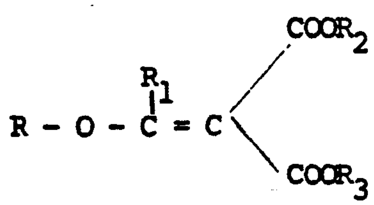

(I)

dans laquelle:

- R représente un radical alkyle comportant de 1 à 4 atomes de carbone et de préférence un radical méthyle ou éthyle;
- $R_1$ représente un atome d'hydrogène, un radical méthyle, éthyle ou phényle;
- $R_2$ et $R_3$ identiques ou différents représentent des radicaux alkyle comportant de 1 à 4 atomes de carbone identiques ou différents de R et de préférence des radicaux méthyle ou éthyle par condensation d'un malonate d'alkyle avec un orthoester en présence d'un acide carboxylique ou de son anhydride et d'un dérivé métallique comme catalyseur caractérisé en ce que ce dernier est choisi dans le groupe formé par les dérivés du cadmium, du magnésium, du bismuth et du mercure.

Depuis les publications de L. CLAISEN, loc. cit., le chlorure de zinc est le catalyseur le plus couramment utilisé parce qu'il conduit aux meilleurs résultats. Dans la demande de brevet japonais n° 4776/66 et dans la demande de brevet français n° 75/17.177 on a bien décrit l'emploi du chlorure d'aluminium et du chlorure ferrique mais ces composés présentent une efficacité moindre que celle du chlorure de zinc; les rendements en alkoxyméthylènemalonates auxquels ils conduisent sont en effet très inférieurs â ceux procurés par le chlorure de zinc. Bien que la demande française précitée se réfère à l'emploi général des acides de Lewis pour la catlyse de la condensation des malonates avec les orthoformiates, la sélection des dérivés de Cd, Hg, Bi et Mg ne pouvait être suggérée par l'art antérieur. On sait en effet que les acides de Lewis regroupent un ensemble vaste de dérivés métalliques dont l'activité catalytique est extrêmement variable d'un métal à l'autre (cf. G. A. OLAH, Friedel-Crafts and Related Reactions, Vol. 1 pages 33 à 34 et pages 169 à 291). Il apparait donc inattendu que parmi un ensemble d'acides de Lewis regroupant des métaux pas actifs ou moins actifs que le chlorure de zinc pour la préparation des alkoxyméthylènemalonates d'alkyle les dérivés de Cd, Hg, Bi et Mg conduisent à des résultats supérieurs ou pratiquement équivalents à ceux du chlorure de zinc.

Comme dérivés de Cd, Hg, Bi et Mg on peut utiliser n'importe quel sel d'acide minéral ou organique tels que les halogénures, sulfates, nitrates, carbonates, phosphates, les carboxylates et les sulfonates.

Pour des raisons pratiques on a recours de préférence aux halogénures et (tout particulièrement aux chlorures et bromures) et aux carboxyletes. Dans ce dernier cas on peut utiliser les sels de Cd, Hg, Bi et Mg de tout acide carboxylique tels que les acides mono- ou polycarboxyliques aliphatiques, cycloaliphatiques saturés ou non ou aromatiques. On peut citer plus particulièrement les sels des acides formique, acétique, propioniques, butyriques, pentanoïques, hexanoïques, octanoïques, dodécanoïques, hexadécanoïques, stéarique, oléïque, ou des mélanges d'acides gras tels que l'acide naphténique, l'acide vendu sous la marque commerciale "acide versatique", l'acide benzoïque. En pratique on a recours de préférence aux chlorures et bromures de Cd, Hg, Bi et Mg et aux carboxylates d'acides aliphatiques inférieurs. A titre non limitatif, on peut citer le chlorure de cadmium, le bromure de cadmium, le chlorure et le bromure de magnésium, le chlorure et le bromure mercurique, le chlorure de bismuth ($BiCl_3$) et le bromure de bismuth ($BiBr_3$), l'acétate de cadmium [$Cd(C_2H_3O_2)_2$], le benzoate de cadmium, l'oxalate de cadmium, le salycilate de cadmium, l'acétate de bismuth [$Bi(C_2H_3O_2)_3$], le benzoate de bismuth, l'acétate mercurique [$Hg(C_2H_3O_2)_2$], le benzoate mercurique, l'oxalate mercurique, l'acétate de magnésium, le benzoate de magnésium, le laurate de magnésium, l'oxalate de magnésium, le palmitate de magnésium, le stéarate de magnésium. En pratique on choisit de préférence les carboxylates de Cd, Hg, Bi et Mg correspondant à l'acide ou à l'anhydride d'acide utilisé comme agent de condensation. Ces sels peuvent être utilisés indifféremment sous forme anhydre ou hydratée. On peut également associer deux ou plus de deux de ces dérivés ou l'un d'entre eux aux composés antérieurement mis en oeuvre comme catalyseurs ($ZnCl_2$, $AlCl_3$, $FeCl_3$).

La quantité de dérivé métallique exprimée en ion-gramme de métal par mole de malonate d'alkyle peut varier entre de larges limites. En pratique cette quantité peut varier entre des limites allant de $1 \times 10^{-5}$ à $1 \times 10^{-2}$ ion-gramme de métal par mole de malonate d'alkyle et de préférence de $1 \times 10^{-4}$ à $1 \times 10^{-3}$ ion gramme par mole.

A titre d'agent de condensation, on utilise préférentiellement les acides carboxyliques aliphatiques et leur anhydride et plus particulièrement les acides aliphatiques inférieurs comportant de 1 à 5 atomes de carbone et leur anhydrides. On peut citer notamment l'acide formique, l'acide acétique, l'acide propionique, l'acide pivalique.

Les catalyseurs selon la présente invention peuvent être utilisés dans les conditions générales de condensation enseignées dans l'art antérieur cité plus haut. Ainsi la température de réaction peut être comprise entre 80 et 180°C et de préférence entre 100 et 170°C; on peut mettre en oeuvre la quantité stoechiométrique d'orthoester ou un excés d'orthoester par rapport au malonate d'alkyle de sorte que la quantité d'orthoester peut varier entre 1 mole et 5 moles par mole de malonate, de préférence entre 1, 2 et 4

moles par mole. La quantité d'acide ou d'anhydride carboxylique peut être inférieure ou égale à 1 mole par mole de malonate, on utilise de préférence une quantité d'acide ou d'anhydride carboxylique allant de 0,01 mole à 0,3 mole par mole de malonate. Dans ce cas l'alcool engendré au cours de la condensation est de préférence éliminé au fur et à mesure de sa formation, par distillation conformément à l'enseignement du brevet américain 2.824.121.

Le procédé selon l'invention se prête particulièrement bien à la préparation des alkoxyméthylènemalonates d'alkyle et notament des methoxy- ou éthoxyméthylènemalonates de méthyle ou d'éthyle par réaction du malonate de méthyle ou d'éthyle avec l'orthoformiate de méthyle ou d'éthyle.

D'un point de vue pratique la totalité des réactifs, du catalyseur et de l'acide carboxylique ou son anhydride peuvent être mis en contact dès le début de la réaction ou, selon une variante d'exécution on peut charger dans un appareil de réaction la totalité du malonate et du catalyseur et une partie de l'orthoester et de l'acide carboxylique ou de son anhydride, porter cette charge à températur adéquate puis ajouter en continu ou par fractions le reste de l'orthoester et de l'acide carboxylique ou de son anhydride au fur et a mesure de l'avancement de la réaction contrôlé par la quantité d'alcool éliminé. La durée de réaction dépend des conditions opératoires mises en oeuvre. Dans certaines conditions il peut être avantageux de maintenir, après disparition du malonate, la masse réactionnelle à la température de réaction pendant une durée nécessaire à la transformation des intermèdiaires de la réaction, en particulier des dialkoxyméthylènemalonates, en alkoxyméthylènemalonates. Ainsi le chauffage pourra être poursuivi jusqu'à l'arrêt de la distillation de l'alcool engendré par la réaction. Lorsque la condensation est achevée, la masse réactionnelle est traitée pour en séparer les différents constitutants: orthoester en excès, alkoxyalkylidènemalonate, acide ou anhydride d'acide et/ou leurs esters, sous-produits de la réaction, dérivé métallique. Les composés organiques sont separes de préférence par distillation. Il est avantageux de séparer le catalyseur métallique de la masse réactionnelle avant de procéder à sa distillation car on a constaté que les dérivés métalliques favorisent la décomposition thermique des alkoxyalkylidènemalonates lors de cette opération. A cet égard l'emploi d'un sel de cadmium s'avère particulièrement avantageux en raison de sa facilité de séparation de la masse réactionnelle: il suffit d'abaisser, en fin de réaction, la température du mélange obtenu pour provoquer la séparation du dérivé métallique au fond du récipient réactionnel, la couche organique supérieure est alors soutirée et le résidu catalytique laissé dans l'appareil de réaction pour une nouvelle opération. Les catalyseurs de l'art antérieur (chlorure de zinc, chlorure d'aluminium, chlorure ferrique) impliquaient le recours à l'élimination du dérivé métallique par extraction à l'eau, procédé qui s'avère plus compliqué et onéreux.

Les exemples suivants illustrent l'invention et montrent comment elle peut être mise en pratique.

## EXEMPLE 1

Dans un ballon en verre de 1 litre a trois tubulures, équipé d'un thermomètre, d'un agitateur central, d'une colonne à distiller, d'une tête de colonne, d'un réfrigérant, d'un séparateur, d'un récepteur et d'un dispositif de chauffage électrique on charge:

| | | |
|---|---|---|
| . malonate de diéthyle | 163,8 | g (1,024 mole) |
| . orthoformiate d'éthyle | 444 | g (3 moles) |
| . acide acetique anhydre | 3 | g (0,05 mole) |
| . Cd $(CH_3COO)_2$, 2 $H_2O$ | 0,134 | g (5 x $10^4$ mole) |

puis on chauffe progressivement le contenu du ballon jusqu'à la température de reflux. On règle la température des vapeurs à environ 78-79° C et soutire les produits volatils de la réaction (éthanol notamment). La distillation commence après 30 minutes de chauffage à 148-151° C.

La temperature de la masse reactionnelle s'élève progressivement de 148 à 157° C en fin de réaction. Après 5 h 30 mn de réaction on dose le malonate de diéthyle (MDE) par chromatographie en phase gazeuse sur une partie aliquote de masse réactionnelle. On constate qu'il reste 0,1 g de MDE. On a recueilli au total 92,8 g de distillat. On maintient la masse réactionnelle encore 2 heures à 157° C. On recueille une quantité supplémentaire de distillat de 9,34 g. Au total on a isolé 102,14 g d'un distillat constitué essentiellement d'éthanol contenant de l'acétate et du formiate de méthyle.

La masse réactionnelle est refroidie à 20° C et on dose par chromatographie en phase gazeuse le malonate de diéthyle (MDE) non transformé, l'orthoformiate d'éthyle (OFE) et l'éthoxyméthylènemalonate d'éthyle (EMME) formé. Les résultats obtenus sont les suivants:

| | |
|---|---|
| MDE | 0,1 g soit un taux de transformation de 100 % |
| OFE | 284,7 g soit un taux de transformation de 35,8 % |
| EMME | 219,3 g. |

Les rendements en EMME par rapport au MDE et à l'OFE transformés (RT) s'élèvent respectivement à 99,2 % et 94,3 %.

**EXEMPLE 2**

On a reproduit l'exemple 1 en utilisant 0,067 g (2,5 x $10^{-4}$ mole) de cadmium. Dans ces conditions on a obtenu des RT par rapport au MDE et à l'OFE s'élèvant respectivement à 98,8 % et 94,4 %.

**EXEMPLES 3 à 5**

On a reproduit l'exemple 1 en remplaçant l'acétate de cadmium par l'acetate mercurique (2,5 x $10^{-4}$ mole), le chlorure de magnésium hexahydraté (1 x $10^{-3}$ mole), le chlorure de bismuth (3 x $10^{-4}$ mole).

On a obtenu les resultats suivants:

| Exemples | TT MDE | TT OFE | EMME dosé en g | RT MDE % | EMME OFE 8% |
|---|---|---|---|---|---|
| 3 | 100 % | 36,2 % | 192,3 | 87 | 82 |
| 4 | 100 % | 34,9 % | 210,9 | 95,3 | 93,2 |
| 5 | 100 % | 34,61 % | 198,6 | 90 | 88,5 |

**EXEMPLE 6**

Dans l'appareil décrit à l'exemple 1 auquel on a adjoint une ampoule de coulée on charge:

| | |
|---|---|
| OFE | 334g |
| MDE | 163,8 g |
| acide acétique | 3g |
| $MgCl_2$, 6 $H_2O$ | 0,218 g |

puis on porte la température du ballon à 151°C en 25 minutes. La réaction débute et l'éthanol formé commence à distiller. On ajoute alors en continu de l'orthofonmiate d'éthyle en quantité équivalente au volum de distillat. La température de la masse réactionnelle se maintient a 155/156°C. Après 3 h 30 mn dans ces condition on a recueili 126 ml de distillat et ajouté 126 ml (110 g) d'OFE. Un dosage effectué sur une partie aliquote de masse réactionnelle montre que la totalite du MDE a été transformée. On maintient la masse réactionnelle pendant 2 heures à 156/157°C, durée pendant laquelle on recueille une quantité supplémentaire de distillat de 10 ml.

La masse réactionnelle est refroidie à 20°C et pesée; son poids s'éléve à 501,49 g. Sur une partie aliquote on dose l'OFE et les produits formés par chromatographie en phase gazeuse. On constate qu'il reste 280,5 g d'OFE et qu'il s'est formé 211,3 g d'EMME.

Les rendements en EMME par rapport au MDE à l'OFE transformés sont respectivement de 95,55 % et 88,5 %.

**Revendications**

1. Procédé de préparation d'alkoxyalkylidènemalonates d'alkyle de formule générale:

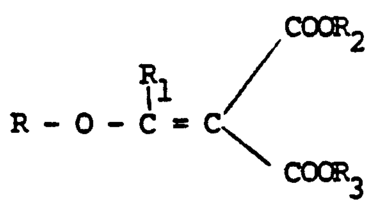

(I)

dans laquelle:

- R représente un radical alkyle comportant de 1 à 4 atomes de carbone;
- $R_1$ représente un atome d'hydrogène, un radical méthyle, éthyle ou phényle;
- $R_2$ et $R_3$ identiques ou différents représentent des radicaux alkyle comportant de 1 à 4 atomes de carbone identiques ou différents de R par condensation d'un malonate d'alkyle avec un orthoester en présence d'un acide carboxylique ou de son anhydride et d'un dérivé métallique comme catalyseur caractérisé en ce que ce

dernier est choisi dans le groupe formé par les dérivés du cadmium, du magnésium, du bismuth et du mercure.

2. Procédé selon la revendication 1, caractérisé en ce qu'on l'applique à la préparation de l'éthoxyméthylènemalonate de diéthyle et du méthoxyméthylènemalonate de diméthyle par réaction des malonates de diéthyle ou de dimétyle avec l'orthoformiate correspondant.

3. Procédé selon l'une quelconque des revendications 1 et 2, caractérisé en ce que l'on utilise comme dérivés de Cd, Hg, Bi et Mg un sel d'acide minéral ou carboxylique.

4. Procédé selon la revendication 3, caractérisé en ce que l'on utilise comme catalyseur l'acétate de cadmium, l'acétate mercurique, le chlorure de magnésium et le chlorure de bismuth.

5. Procédé selon l'une quelconque des revendications 1 à 4, caractérisé en ce que la qrantité de catalyseur exprimée en ion-gramme de métal par mole de malonate est comprise entre $1 \times 10^{-5}$ et $1 \times 10^{-2}$ ion-gramme par mole.

6. Procédé selon l'une quelconque des revendications 1 à 5, caractérisé en ce que la quantité d'acide carboxylique ou d'anhydcide est comprise entre 0,01 et 1 mole par mole de malonate.

7. Procédé selon l'une quelconque des revendications 1 à 6, caractérisé en ce que la quantité d'orthoester est comprise entre 1 et 5 moles par mole de malonate.

8. Procédé selon l'une quelconque des revendications 1 à 7, caractérisé en ce que la température de la réaction est comprise entre 80 et 180° C.

## Patentansprüche

1. Verfahren zur Herstellung alkylierter Alkoxyalkylidenmalonate der allgemeinen Fomel:

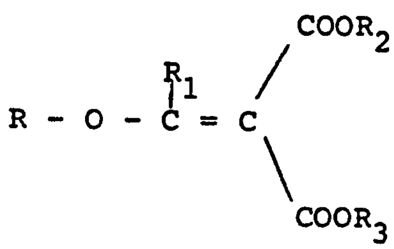

(I)

in der:

- R einen Alkylrest mit 1 bis 4 Kohlenstoffatomen darstellt;
- $R_1$ ein Wasserstoffatom, einen Methyl-, Ethyl- oder Phenylrest darstellt;
- $R_2$ und $R_3$ gleich oder unterschiedlich sind und Alkylreste darstellen mit 1 bis 4 Kohlenstoffatomen, die gleich R oder von R verschieden sind,

durch Kondensation eines Alkylmalonats mit einem Orthoester in Gegenwart einer Carbonsäure oder deren Anhydrid und eines metallischen Derivats als Katalysator, dadurch gekennzeichnet, daß letzteres ausgewählt wird aus der Gruppe umfassend die Verbindungen des Cadmiums, Magnesiums, Wismuts und des Quecksilbers.

2. Verfahren nach Anspruch 1, dadurch gekennzeichnet, daß man dieses auf die Herstellung des (Diethyl)ethoxymethylenmalonats und des (Dimethyl)methoxymethylenmalonats durch Reaktion der Diethylmalonate oder Dimethylmalonate mit dem entsprechenden Orthoformiat anwendet.

3. Verfahren nach einem der Ansprüche 1 und 2, dadurch gekennzeichnet, daß man als Verbindungen des Cd, Hg, Bi und Mg ein Salz einer Mineralsäure oder Carbonsäure verwendet.

4. Verfahren nach Anspruch 3, dadurch gekennzeichnet, daß man als Katalysator Cadmiumacetat, Quecksilberacetat, Magnesiumchlorid und Wismutchlorid verwendet.

5. Verfahren nach einem der Ansprüche 1 bis 4, dadurch gekennzeichnet, daß die Menge an Katalysator ausgedrückt in Ionen-Gramm des Metalls pro Mol Malonat zwischen $1 \times 10^{-5}$ und $1 \times 10^{-2}$ Ionen-Gramm pro Mol liegt.

6. Verfahren nach einem der Ansprüche 1 bis 5, dadurch gekennzeichnet, daß die Menge der Carbonsäure oder des Anhydrids zwischen 0,01 und 1 Mol pro Mol Malonat liegt.

7. Verfahren nach einem der Ansprüche 1 bis 6, dadurch gekennzeichnet, daß die Menge des Orthoesters zwischen 1 und 5 Mol pro Mol Malonat liegt.

8. Verfahren nach einem der Ansprüche 1 bis 7, dadurch gekennzeichnet, daß die Reaktionstemperatur zwischen 80° und 180° C liegt.

## Claims

1. Process for preparing alkyl alkoxyalkylidenemalonates of general formula:

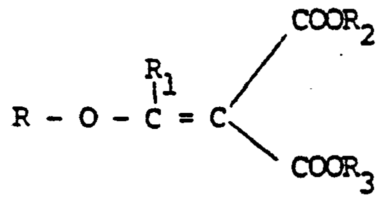

(I)

in which:

R denotes an alkyl radical containing from 1 to 4 carbon atoms;

$R_1$ denotes a hydrogen atom or a methyl, ethyl or phenyl radical; and

$R_2$ and $R_3$, which may be identical or different, denote alkyl radicals containing from 1 to 4 carbon atoms which may be identical to or different from R, by condensation of an alkyl malonate with an orthoester in the presence of a carboxylic acid or its anhydride, and a metal derivative as catalyst, characterized in that the latter is chosen from the group consisting of cadmium, magnesium, bismuth and mercury derivatives.

2. Process according to Claim 1, characterized in that it is applied to the preparation of diethyl ethoxymethylenemalonate and dimethyl methoxymethylenemalonate by reaction of diethyl or dimethyl malonate with the corresponding orthoformate.

3. Process according to any one of Claims 1 and 2, characterized in that a salt of an inorganic or carboxylic acid is used as Cd, Hg, Bi and Mg derivatives.

4. Process according to Claim 3, characterized in that cadmium acetate, mercuric acetate, magnesium chloride and bismuth chloride are used as catalyst.

5. Process according to any one of Claims 1 to 4, characterized in that the amount of catalyst, expressed as gram-ion of metal per mole of malonate, is between $1 \times 10^{-5}$ and $1 \times 10^{-2}$ gram-ion per mole.

6. Process according to any one of Claims 1 to 5, characterized in that the amount of carboxylic acid or anhydride is between 0.01 and 1 mole per mole of malonate.

7. Process according to any one of Claims 1 to 6, characterized in that the amount of orthoester is between 1 and 5 moles per mole of malonate.

8. Process according to any one of Claims 1 to 7, characterized in that the temperature of the reaction is between 80 and 180° C.